# EUROPÄISCHE PATENTSCHRIFT

(11) **EP 2 086 522 B1**
(45) Veröffentlichungstag und Bekanntmachung des Hinweises auf die Patenterteilung: **13.04.2016**
(21) Anmeldenummer: 07818870.3
(22) Anmeldetag: 10.10.2007
(51) Int. Cl.: A61K 9/70, A61K 31/57

(54) **TRANSDERMALES THERAPEUTISCHES SYSTEM ENTHALTEND NORELGESTROMIN ZUR KONTRAZEPTION UND HORMONSUBSTITUTION**
TRANSDERMAL THERAPEUTIC SYSTEM COMPRISING NORELGESTROMIN FOR CONTRACEPTION AND HORMONE REPLACEMENT
SYSTÈME THÉRAPEUTIQUE TRANSDERMIQUE CONTENANT DE LA NORELGESTROMINE POUR LA CONTRACEPTION ET LA SUBSTITUTION HORMONALE

(30) Priorität: 26.10.2006 DE 102006050558
(43) Veröffentlichungstag der Anmeldung: 12.08.2009
(73) Patentinhaber: LTS LOHMANN Therapie-Systeme AG, 56626 Andernach (DE)
(72) Erfinder: THEOBALD, Frank, 56653 Wehr (DE); EIFLER, René, 56072 Koblenz (DE)
(74) Vertreter: Siemund, Volker
(86) Internationale Anmeldenummer: PCT/EP2007/008797
(87) Internationale Veröffentlichungsnummer: WO 2008/049516

(56) Entgegenhaltungen:
- WO-A-2006/036899
- DE-A1- 4 332 094
- DE-A1-102006 026 060
- CENTER FOR DRUG EVALUATION AND RESEARCH: "Ortho Evra (Norelgestromin/ Ethinyl Estradiol Transdermal System)" NDA, CENTER FOR DRUG EVALUATION AND RESEARCH, US, Nr. NDA21-180, 20. November 2001 (2001-11-20), XP002346644

## Beschreibung

Die vorliegende Erfindung betrifft ein transdermales therapeutisches System zur Applikation des Wirkstoffs Norelgestromin an die Haut, optional in Kombination mit Estrogenen. Sie betrifft ferner die Verwendung derartiger Systeme zur hormonalen Kontrazeption, sowie zur Hormonsubstitutionstherapie.

Der Wirkstoff Norelgestromin (17-Deacylnorgestimat; 13-Ethyl-17-hydroxy-18,19-dinor-17-alpha-pregn-4-en-20-yn-3-on oxim) gehört als Sexualhormon zu der Klasse der Gestagene. Norelgestromin hemmt die Freisetzung des lutenisierenden Hormons (LH) und wirkt so ovulationshemmend. Vorteilhaft an Norelgestromin ist seine geringere androgene Wirkung im Vergleich zum Prodrug Norgestimat oder dessen anderen Metaboliten, wie 3-Ketonorgestimat und Levonorgestrel. Estrogene inhibieren die Sekretion des follikelstimmulierenden Hormons (FSH) und bewirken so eine Hemmung der Ovulation. Zu den Estrogenen zählen beispielsweise 17-beta-Estradiol und Ethinylestradiol. Präparate mit Norelgestromin, wie auch Kombinationspräparate mit Norelgestromin und Ethinylestradiol werden zur Kontrazeption sowie zur Hormonsubstitutionstherapie bei Frauen eingesetzt.

Die Kontrazeption stellt keine Erkrankung im klassischen Sinne dar, da sie ja nicht der Behandlung eines lebensbedrohlichen oder behandlungsbedürftigen Krankheitszustandes dient. Deshalb wünschen sich viele Frauen eine Arzneiform, die eine diskrete Form der Applikation ermöglicht und, wenn möglich, die tägliche Anwendung, wie sie bei den oralen Kontrazeptiva erforderlich ist, vermeidet. So sind im Bereich der Kontrazeption Implantate, Vaginalringe aber auch transdermale Systeme vorzufinden, die eine weitgehend unauffällige Applikation ermöglichen. Nachteilig bei vielen neuartigen Applikationsformen wie z.B. Implantaten, Vaginalringen etc. wirkt sich aus, dass die Anwenderin häufig nur mangelndes Zutrauen zu solchen Arzneiformen hat, weil man im Gegensatz zu Tabletten nicht sicher sein kann, dass man die Arzneiform ordnungsgemäß appliziert hat. Dieses Gefühl der Sicherheit ist insbesondere in der Kontrazeptionstherapie für die Anwenderin sehr wichtig und beeinflusst die Compliance signifikant.

Transdermale Systeme erlauben eine sehr diskrete Anwendung und zudem eine visuelle Kontrolle der korrekten Applikation im Zeitraum der Anwendung. Der Erfolg des Ortho Evra™ TCS (transdermal contraceptive system, Norelgestromin/Ethinyl estradiol) zeigt, dass Frauen diese Form der Applikation gut annehmen.
Eine Vielzahl von Transdermalen Therapeutischen Systemen (TTS) zur Verabreichung dieser Wirkstoffe ist im Stand der Technik bereits bekannt. Derartige TTS weisen typischerweise einen Aufbau aus einer arzneistoffundurchlässigen Trägerschicht, einer arzneistoffhaltigen Reservoirschicht, sowie einer Haftkleberschicht zur Befestigung auf der Haut auf, wobei letztere mit der arzneistoffhaltigen Reservoirschicht identisch sein kann. Die arzneistoffhaltige Schicht kann noch weitere Inhaltsstoffe enthalten, z. B. Weichmacher, Klebrigmacher, Lösungsvermittler, Stabilisatoren, Füllstoffe, Trägerstoffe und Permeationsbeschleuniger. Die hierfür in Frage kommenden pharmazeutisch unbedenklichen Substanzen sind dem Fachmann grundsätzlich bekannt.

Auch wenn Transdermale Therapeutische Systeme als Darreichungsformen grundsätzlich bekannt sind, stellt die Formulierung eines bestimmten Wirkstoffs, z. B. Norelgestromin als TTS eine Herausforderung dar. Es können unterschiedliche Probleme auftreten. So muss ein TTS, um therapeutisch eingesetzt werden zu können, einen genügend hohen Wirkstoff-Flux durch die Haut ermöglichen. Es muss ferner eine gute Stabilität aufweisen und darf insbesondere während der Lagerung keinen Veränderungen unterliegen. Die Auswahl geeigneter Polymere für das Wirkstoffreservoir kann sich als schwierig erweisen, da diese Polymere kompatibel zu dem jeweiligen Wirkstoff sein müssen. Eine weitere Anforderung an das erfindungsgemäße TTS ist, dass es kostengünstig hergestellt werden kann.

Ein solches System ist zum Beispiel in US 6,071,531 beschrieben. Die dort beschriebene Formulierung besteht aus einem nicht Acrylat-basierten Polymer, einem Enhancer und einem Lösungsmittel, wobei der Enhancer aus der Gruppe der Lactatester mit C₁₂-C₁₈ Grundgerüst der aliphatischen Alkohole, Propylengylocolmonolaurat und Kombinationen dieser Stoffe besteht. Das kommerziell in vielen Ländern der Welt erhältliche Ortho Evra™ TCS nutzt z. B. die Lehre dieses Patents.

Die WO 2005/120470 A1 offenbart ein matrixkontrolliertes transdermales therapeutisches System mit Norelgestromin allein oder in Kombination mit einem Östrogen, insbesondere Ethinylestradiol. Für die Matrix wird ein Schmelzklebstoff, insbesondere ein Styrol-Block-Copolymer, verwendet.

Nachteilig bei dem auf dem Markt befindlichen System (Ortho Evra™ TCS) ist seine verhältnismäßig große Applikationsfläche von 20 cm², die die Anwendung erschwert.

WO9640355 offenbart Formulierungen Für TTS zur hormonellen Verabreichung von östrogenen und Progestinen.

Aufgabe der vorliegenden Erfindung war es deshalb, die genannten Nachteile im Stand der Technik zu vermeiden und ein transdermales therapeutisches System zur Verabreichung von Norelgestromin, optional in Kombination mit Estrogen bereitzustellen, das durch eine erhöhte Abgaberate des/der Wirkstoff(e)s zur Kontrazeption oder zur Hormonsubstitutionstherapie besser geeignet ist. Dabei soll eine verbesserte Patienten-Compliance durch eine einfachere und sichere, Applikationsmethode, erreicht werden. Die Anwendung sollte darüber hinaus möglichst diskrete sein.

Erfindungsgemäß wird diese Aufgabe durch ein transdermales therapeutisches Systems (TTS) gemäß Anspruch 1 gelöst, welches ein Norelgestromin enthaltendes, mindestens einschichtiges Wirkstoffreservoir auf der Basis von Silikonpolymeren aufweist. Überraschend hat sich gezeigt, dass insbesondere die Kombination von Norelgestromin und Ethinylestradiol mit einem Stoff oder einer Kombination von Stoffen wie Diethylenglycolmonoethlyether, Butandiol oder 1,2-Propylenglykol eine wesentlich höhere Permeation durch die Haut erzielt werden kann, als im Vergleich zu dem auf dem Markt befindlichen Ortho Evra™ TCS. Die erfindungsgemäße Formulierung zeigt deutlich höhere Permeations-Werte durch Humanhaut-Epidermis über einen vorgegebenen Zeitraum von 72 Stunden. Die TTS Formulierungen weist dabei unterschiedliche Gehalte an Norelgestromin und Ethinylestradiol auf.

Das erfindungsgemäße TTS kann nach Applikation auf die Haut über einen längeren Zeitraum, z. B. bis zu 7 Tagen, verbleiben und während dieser Zeit den/die Wirkstoff(e) an die Haut in kontrollierter Weise abgeben. Auf diese Weise wird die Notwendigkeit einer täglichen Applikation, wie dies bei oralen Kontrazeptiva der Fall ist, vermieden. Falls erforderlich, kann die Behandlung auf einfache Weise durch Entfernen des TTS von der Haut abgebrochen bzw. unterbrochen werden.

Ein besonderer Vorteil ist, dass auf diese Weise TTS mit deutlich kleineren Applikationsflächen, verglichen mit Applikationsflächen von Ortho Evra™ TCS, bei gleichen Permeationsraten um 17 %, bevorzugt um 36 %, besonders bevorzugt um 48 % eingesetzt werden können. Ein leichteres Handling und verbesserter Tragekomfort ist damit verbunden.

Die Zugabe der o.g. Stoffe bewirkt, dass zwischen der Silikonmatrix und dem Wirkstoff eine Lösungsvermittlung erzeugt wird. Bevorzugt ist die Verwendung von Diethylenglycolmonoethlyether. Als besonders vorteilhaft hat sich herausgestellt, wenn der Anteil der Substanzen, bezogen auf das Matrixgewicht von 1 bis 80 Gew.-%, vorzugsweise zwischen 5 und 40 Gew.-% beträgt.

Als geeignete Silikonpolymere kommen Silikone, Polysiloxane oder Silikone, die selbst haftklebend sein können, zur Anwendung. Als Silikonhaftkleber werden Haftkleber z.B. basierend auf einer Polydimethylsiloxanstruktur oder Polydimethyldiphenylsiloxanstruktur verstanden. Insbesondere kommerziell erhältliche SilikonHaftkleber, wie z.B. BIO-PSA von Dow Corning Corporation sind geeignet. Bevorzugt wird BIO-PSA Q7-4302 eingesetzt.

Üblicherweise umfasst ein TTS eine wirkstoffundurchlässige Rückschicht, eine oder mehre wirkstoffhaltige Matrixschichten, optional eine die Wirkstofffreisetzung steuernde Membran und eine abziehbare Schutzschicht, wobei die Matrixschicht selbstklebend ausgerüstet oder zusätzlich mit einer hautseitigen Haftkleberschicht versehen sein kann.

Das Wirkstoffreservoir ist vorzugsweise aus einer mindestens einschichtigen Polymermatrix aufgebaut, welche den Wirkstoff Norelgestromin und optional weitere Wirkstoffe, wie z. B. Estradiol, Ethinylestradiol oder Estriol enthält. Vorteilshafterweise können noch Wirkstoffe mit agonistischer Wirkung am Estradiolrezeptor enthalten sein. Das transdermale therapeutische System kann auch aus mehreren wirkstoffhaltigen Matrixschichten bestehen, die sich in ihrer Zusammensetzung und/oder im Wirkstoffgehalt unterscheiden können.

Die Wirkstoffmatrix kann neben den genannten Polymeren und Wirkstoffen zusätzlich noch weitere Hilfsstoffe, wie Weichmacher, klebrigmacher, Lösungsvermittler, Stabilisatoren, Füllstoffe, Trägerstoffe, PermeationsBeschleuniger enthalten, die dem Fachmann grundsätzlich bekannt sind.

Beispielhaft seien hier in weiteren bevorzugten Ausführungsformen noch eine oder mehrere Fettsäuresalze in der Wirkstoffmatrix, bevorzugt Natriumlaurat, oder weitere Polymere, wie zum Beispiel Ethyl-, Methylcellulose, Polyvinylpyrrolidon, Tragant, Bentonit, Lactose, hochdisperses Siliciumdioxid aufgeführt. Die Mengen liegen von 1 bis 50 Gew.-%, vorzugsweise zwischen 2 und 15 Gew.-%.

Weitere bevorzugte Hilfsstoffe der vorliegenden Erfindung sind z. B. Silikonöl, Glycerinester von hydrierten Harzsäuren, Hydroabietin-Alkohol-Harz-Ester, Hydroabietin-Säure-Harz-Ester, hydrogenierte Methylester von Terpentinharzen, Ester von partiell hydrierten Terpentinharzen, Ester von Terpentinharzen.

Alternativ können zusätzlich noch Antioxidantien, wie Tocopherole, Butylhydroxyanisol (BHA), Gallussäureester, Butylhydroxytoluol (BHT), Ascorbylpalmitat, -stearat, zur Stabilisierung bzw. Minimierung des Wirkstoffabbaus während der Lagerung eingesetzt werden.

Als Rückschicht eigenen sich insbesondere Polyester, wie z. B. bevorzugt Polyethylenterephthalat (PET) und Polybutylenterephthalat, welche sich durch besondere Festigkeit auszeichnen. Darüber hinaus sind nahezu beliebige andere verträgliche Kunststoffe, wie z.B. Polyvinylchlorid, Ethylenvinylacetat, Vinylacetat, Polyethylen, Polypropylen, Cellulosederivate oder Kombinationen verschiedener Folien geeignet.

Besonders bevorzugt sind erfindungsgemäße Wirkstoffpflaster, die aus einer wirkstoffhaltigen Matrix und darüber liegender Rückschicht aufgebauten sind, wobei die resultierenden TTS nach Applikation auf der Haut transparent oder zumindest transluzent (d. h. durchscheinend) sind.

Auch kann es hilfreich sein, zur mechanischen Stabilisierung des TTS zusätzlich eine Stützschicht z.B. ein Vlies einzusetzen, wobei Vorteilhafterweise der in einem Lösemittel gelöste oder suspendierte Wirkstoff auf das Vlies aufgebracht ist. Geeignete Materialien als Vliesstoffe sind dem Fachmann bekannt, bevorzugt Polyethylenterephthalat, Cellulose, regenerierte Cellulose, Cellulosenitrat oder Polyethylen.

Die erfindungsgemäßen transdermalen therapeutischen Systeme finden zur hormonalen Kontrazeption, sowie zur Hormonsubstitutionstherapie Anwendung.

Die Erfindung wird durch nachstehendes Beispiel näher erläutert, ohne aber den Erfindungsumfang damit einzuschränken.

Es wurde eine Formulierung im Labormaßstab hergestellt und hinsichtlich Ihrer Humanhautpermeation untersucht:

| **Zusammensetzung bezogen auf die Matrix** | **Gew.-%** |
|---|---|
| Norelgestromin | 3,50 % |
| Ethinylestradiol | 0,50 % |
| DL-α-Tocopherol | 0,40 % |
| BIO PSA Q7-4302 | 82,60 % |
| Kollidon 90 F USP | 3,00 % |
| Natriumlaurat | 2,00 % |
| Diethylenglycolmonoethylether | 8,00 % |

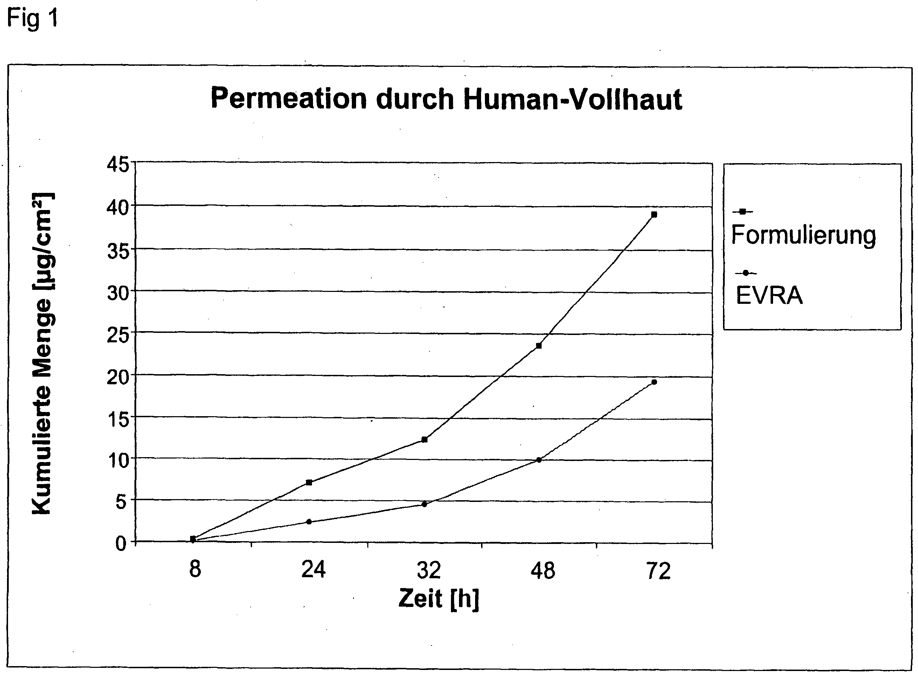

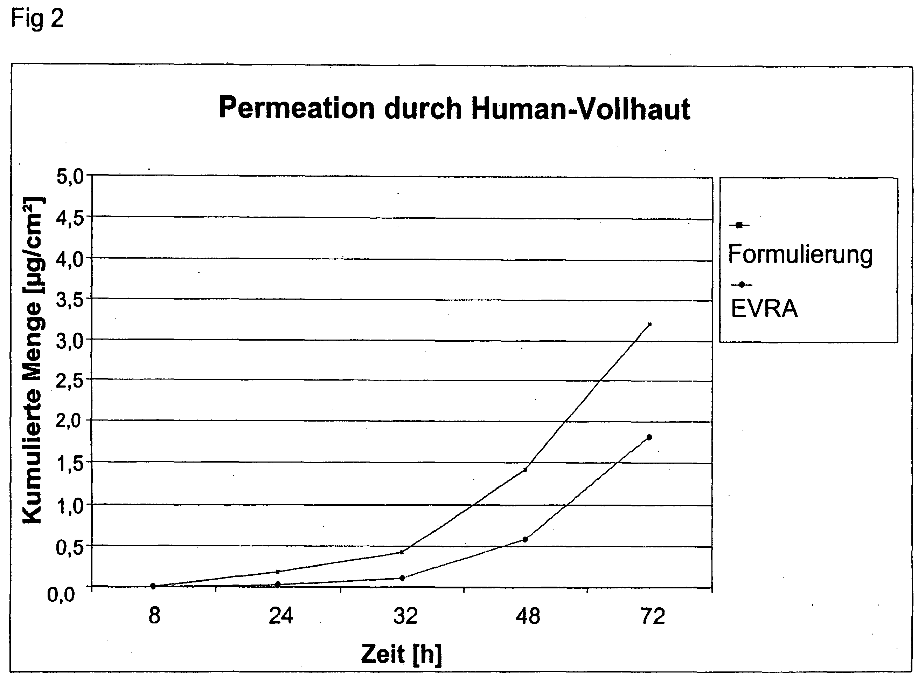

Die Permeation durch Humanhaut-Epidermis für Norelgestromin ist in Figur 1 dargestellt.
Die Permeation durch Humanhaut-Vollhaut für Ethinylestradiol ist in Figur 2 dargestellt.

Die Formulierung zeigt in etwa doppelt so gute Permeationsergebnisse im Vergleich zu dem auf dem Markt befindlichen Ortho Evra™ TCS.

## Patentansprüche

1. Transdermales therapeutisches System zur Applikation von Norelgestromin umfassend eine wirkstoffundurchlässige Rückschicht, zumindest eine wirkstoffhaltige Matrixschicht, optional eine ablösbare Schutzschicht, wobei die Matrixschicht zumindest enthält:
a) eine therapeutisch wirksame Dosis an Norelgestromin und einen Wirkstoff oder seine Derivate ausgewählt aus der Gruppe bestehend aus Estradiol, Ethinylestradiol, Estriol
b) ein oder mehrere Silikonpolymere,
c) ein oder mehrere Stoffe ausgewählt aus der Gruppe bestehend aus Diethylenglykolmonoethylether, Butandiol und 1,2-Propandiol.
d) eine oder mehrere Fettsäuresalze.

2. Transdermales therapeutisches System gemäß Anspruch 1, **dadurch gekennzeichnet, dass** es zusätzlich ein Wirkstoff mit agonistischer Wirkung am Estradiolrezeptor enthält.

3. Transdermales System nach Anspruch 1, **dadurch gekennzeichnet, dass** es als Fettsäuresalz Natriumlaurat enthält.

4. Transdermales System nach einem oder mehreren der vorangehenden Ansprüche, **dadurch gekennzeichnet, dass** es zusätzlich ein Polymer oder Kombinationen solcher Polymere ausgewählt aus der Gruppe umfassend Ethyl-, Methylcellulose, Polyvinylpyrrolidon und Lactose enthält.

5. Transdermales System nach einem oder mehreren der vorangehenden Ansprüche, **dadurch gekennzeichnet, dass** es zusätzlich eine oder mehrere Stoffe oder deren Kombinationen ausgewählt aus der Gruppe umfassend Silikonöl, Glycerinester hydrierter Harzsäuren, Hydroabietin-Alkohol-Harz-ester, Hydroabietin-Säure-Harz-ester, hydrogenierte Methylester von Terpentinharzen, Ester von partiell hydrierten Terpentinharzen und Ester von Terpentinharzen enthält.

6. Transdermales System nach einem oder mehreren der vorangehenden Ansprüche, **dadurch gekennzeichnet, dass** es zusätzlich ein Stoff oder Kombinationen solcher Stoffe ausgewählt aus der Gruppe umfassend Tragant, Bentonit, Lactose, hochdisperses Siliciumdioxid enthält.

7. Transdermales System nach einem oder mehreren der vorangehenden Ansprüche, **dadurch gekennzeichnet, dass** es zusätzlich ein oder mehrere Antioxidantien enthält.

8. Transdermales therapeutisches System nach einem oder mehreren der vorangehenden Ansprüche, **dadurch gekennzeichnet, dass** es transparent bzw. transluzent ist.

9. Transdermales System nach einem oder mehreren der vorangehenden Ansprüche, **dadurch gekennzeichnet, dass** es als Stützschicht zusätzlich ein Vlies enthält.

10. Transdermales System nach Anspruch 9 **dadurch gekennzeichnet, dass** der in einem Lösemittel gelöste oder suspendierte Wirkstoff oder eine Kombination von Wirkstoffen auf das Vlies aufgebracht ist.

11. Transdermales System nach Anspruch 9 oder 10 **dadurch gekennzeichnet, dass** das Vlies aus Polyethylenterephthalat, Cellulose, regenerierte Cellulose, Cellulosenitrat oder Polyethylen besteht.

12. Transdermales therapeutisches System nach einem oder mehreren der vorangehenden Ansprüche, **dadurch gekennzeichnet, dass** es aus mehreren Schichten besteht, die sich in ihrer Zusammensetzung und/oder im Wirkstoffgehalt unterscheiden.

13. Verwendung eines transdermalen therapeutischen Systems nach einem oder mehreren der vorangehenden Ansprüche zur Herstellung eines Arzneimittels zur hormonellen Kontrazeption.

14. Verwendung eines transdermalen therapeutischen Systems nach einem oder mehreren der vorangehenden Ansprüche zur Herstellung eines Arzneimittels zur Hormonsubstitution.

## Claims

1. Transdermal therapeutic system for administration of norelgestromin comprising an active ingredient-impermeable backing layer, at least one active ingredient-containing matrix layer, optionally a detachable protective layer, where the matrix layer comprises at least:
a) a therapeutically effective dose of norelgestromin and an active ingredient or its derivatives selected from the group consisting of estradiol, ethinylestradiol, estriol,
b) one or more silicone polymers,
c) one or more substances selected from the group consisting of diethylene glycol monoethyl ether, butanediol and 1,2-propanediol,
d) one or more fatty acid salts.

2. Transdermal therapeutic system according to Claim 1, **characterized in that** it additionally comprises an active ingredient having an agonistic effect on the estradiol receptor.

3. Transdermal system according to Claim 1, **characterized in that** it comprises sodium laurate as fatty acid salt.

4. Transdermal system according to one or more of the preceding claims, **characterized in that** it additionally comprises a polymer or combinations of such polymers selected from the group comprising ethyl-, methylcellulose, polyvinylpyrrolidone and lactose.

5. Transdermal system according to one or more of the preceding claims, **characterized in that** it additionally comprises one or more substances or combinations thereof selected from the group comprising silicone oil, glycerol esters of hydrogenated resin acids, hydroabietic alcohol resin esters, hydroabietic acid resin esters, hydrogenated methyl esters of terpentine resins, esters of partially hydrogenated terpentine resins and esters of terpentine resins.

6. Transdermal system according to one or more of the preceding claims, **characterized in that** it additionally comprises a substance or combinations of such substances selected from the group comprising tragacanth, bentonite, lactose, colloidal silicon dioxide.

7. Transdermal system according to one or more of the preceding claims, **characterized in that** it additionally comprises one or more antioxidants.

8. Transdermal therapeutic system according to one or more of the preceding claims, **characterized in that** it is transparent or translucent.

9. Transdermal system according to one or more of the preceding claims, **characterized in that** it additionally comprises a nonwoven supporting layer.

10. Transdermal system according to Claim 9, **characterized in that** the active ingredient dissolved or suspended in a solvent, or a combination of active ingredients, is applied to the nonwoven.

11. Transdermal system according to Claim 9 or 10, **characterized in that** the nonwoven consists of polyethylene terephthalate, cellulose, regenerated cellulose, cellulose nitrate or polyethylene.

12. Transdermal therapeutic system according to one or more of the preceding claims, **characterized in that** it consists of a plurality of layers which differ in their composition and/or active ingredient content.

13. Use of a transdermal therapeutic system according to one or more of the preceding claims for producing a medicinal product for hormonal contraception.

14. Use of a transdermal therapeutic system according to one or more of the preceding claims for producing a medicinal product for hormone replacement.

## Revendications

1. Système thérapeutique transdermique pour l'application de norelgestromine, comprenant une couche arrière imperméable à l'agent actif, au moins une couche de matrice contenant l'agent actif, éventuellement une couche de protection détachable, la couche de matrice contenant au moins :
a) une dose thérapeutiquement efficace de norelgestromine et un agent actif ou son dérivé choisi dans le groupe constitué par l'estradiol, l'éthynylestradiol, l'estriol,
b) un ou plusieurs polymères de silicone,
c) une ou plusieurs substances choisies dans le groupe constitué par l'éther monoéthylique de diéthylène glycol, le butanediol et le 1,2-propanediol,
d) un ou plusieurs sels d'acides gras.

2. Système thérapeutique transdermique selon la revendication 1, **caractérisé en ce qu'**il contient en outre un agent actif à effet agoniste sur le récepteur d'estradiol.

3. Système transdermique selon la revendication 1, **caractérisé en ce qu'**il contient du laurate de sodium en tant que sel d'acide gras.

4. Système transdermique selon une ou plusieurs des revendications précédentes, **caractérisé en ce qu'**il contient en outre un polymère ou des combinaisons de tels polymères choisis dans le groupe comprenant l'éthyl-, la méthylcellulose, la polyvinylpyrrolidone et le lactose.

5. Système transdermique selon une ou plusieurs des revendications précédentes, **caractérisé en ce qu'**il contient en outre une ou plusieurs substances ou leurs combinaisons choisies dans le groupe comprenant l'huile de silicone, les esters de glycérine d'acides résiniques hydrogénés, les esters d'hydroabiétine-alcool-résine, les esters d'hydroabiétine-acide-résine, les esters méthyliques hydrogénés de résine de térébenthine, les esters de résines de térébenthine partiellement hydrogénées et les esters de résines de térébenthine.

6. Système transdermique selon une ou plusieurs des revendications précédentes, **caractérisé en ce qu'**il contient en outre une substance ou des combinaisons de telles substances choisies dans le groupe comprenant la tragacanthe, la bentonite, le lactose, le dioxyde de silicium hautement dispersé.

7. Système transdermique selon une ou plusieurs des revendications précédentes, **caractérisé en ce qu'**il contient en outre un ou plusieurs antioxydants.

8. Système transdermique selon une ou plusieurs des revendications précédentes, **caractérisé en ce qu'**il est transparent ou translucide.

9. Système transdermique selon une ou plusieurs des revendications précédentes, **caractérisé en ce qu'**il contient en outre un non-tissé en tant que couche support.

10. Système transdermique selon la revendication 9, **caractérisé en ce que** l'agent actif ou une combinaison d'agents actifs dissous ou suspendu dans un solvant est appliqué sur le non-tissé.

11. Système transdermique selon la revendication 9 ou 10, **caractérisé en ce que** le non-tissé est constitué de polyéthylène téréphtalate, de cellulose, de cellulose régénérée, de nitrate de cellulose ou de polyéthylène.

12. Système thérapeutique transdermique selon une ou plusieurs des revendications précédentes, **caractérisé en ce qu'**il est constitué par plusieurs couches qui diffèrent au niveau de leur composition et/ou de leur teneur en agent actif.

13. Utilisation d'un système thérapeutique transdermique selon une ou plusieurs des revendications précédentes pour la fabrication d'un médicament pour la contraception hormonale.

14. Utilisation d'un système thérapeutique transdermique selon une ou plusieurs des revendications précédentes pour la fabrication d'un médicament pour la substitution hormonale.
